# Europäisches Patentamt

## European Patent Office

## Office européen des brevets

(11) Publication number: **0 040 512**
**B1**

(12) **EUROPEAN PATENT SPECIFICATION**

(45) Date of publication of patent specification: **08.01.86**

(21) Application number: **81302134.2**

(22) Date of filing: **13.05.81**

(51) Int. Cl.⁴: **A 61 N 1/40,** H 01 F 1/113,
H 05 B 6/02, A 61 K 9/00

(54) Ceramic suitable for inducing localised heating in the presence of a radio frequency magnetic field, and use thereof.

(30) Priority: **19.05.80 US 151210**

(43) Date of publication of application:
**25.11.81 Bulletin 81/47**

(45) Publication of the grant of the patent:
**08.01.86 Bulletin 86/02**

(84) Designated Contracting States:
**BE DE FR GB IT NL**

(56) References cited:
**DE-B-1 284 528**
**US-A-4 042 519**
**US-A-4 084 973**
**US-A-4 106 488**
**US-A-4 140 645**

**IEEE TRANSACTIONS ON BIO-MEDICAL
ENGINEERING, vol. BME-13, no. 3, July 1966,
New York, USA C.V. BURTON et al. "Induction
thermocoagulation of the brain: a new
neurosurgical tool". pages 114-120.**

(73) Proprietor: **Corning Glass Works
Houghton Park
Corning New York 14831 (US)**

(72) Inventor: **Borrelli, Nicholas Francis
935 West Water Street
Elmira New York (US)**
Inventor: **Luderer, Albert August
Goff Road
Corning New York (US)**
Inventor: **Panzarino, Joseph Nicholas
32 Downing Street
Big Flats New York (US)**

(74) Representative: **Smith, Sydney et al
Elkington and Fife High Holborn House 52/54
High Holborn
London WC1V 6SH (GB)**

(56) References cited:
**ELECTROCOMPONENT SCIENCE AND
TECHNOLOGY, vol. 1, no. 2, December 1974,
London, GB P.C. THACKRAY et al. "Indirect
heating source for treatment of malignant
brain tumors", pages 91-96.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

This invention relates to a material suitable for including localised heating in the presence of a radio frequency magnetic field, and to the use thereof.

It has been recognised that, when heat is applied to areas of animal tissue containing both normal and malignant cells sufficient to raise the temperature of such areas to the range of from 41 to 44°C, a preferential destruction of the malignant cells occurs. (Normal animal tissue is destroyed at a temperature of about 48°C). Examinations of tumours subjected to such heat treatments utilising light microscope and electron microscope techniques have revealed that the tumours undergo specific destruction with no substantial damage to adjacent normal cells, such as fibroblasts and endothelial cells. The initial result of hyperthermia applied to solid *in vivo* tumours is the rapid increase of lysosomal enzyme activity in the cytoplasm of maligant cells with concomitant inhibition of respiratory metabolism. Significantly, a simultaneous depression of anaerobic glycolysis does not take place in the malignant cells, thereby promoting the accumulation of lactic acid first in the intra-cellular spaces and subsequently in the extra-cellular spaces. Inasmuch as most solid tumours exhibit slow exchange between intra-cellular fluid and blood, this circumstance being particularly true in the central tumour regions, acidic conditions become predominant within the tumour during hyperthermia. This increase in acidity leads directly to enhanced lysosomal enzymatic activity (pH maxima from 5 to 5.5). The non-malignant cells surrounding the tumour sustain only minor and reversible damage. Both malignant and normal tissue demonstrate a rapid and marked inhibition of RNA synthesis which is subsequently followed by the partial inhibition of DNA and protein synthesis. A transitory effect of cell proliferation has also been observed. Nevertheless, these adverse effects are customarily eclipsed by the highly desirable rapid and pronounced lysosomal destruction occurring preferentially in the malignant cells. The biochemical lesion(s) affecting both RNA and DNA metabolism and the cells' ability to divide appear to be transient and are not believed to be the primary cause of hyperthermia-induced destruction. It has been postulated that a significant factor in normal cell survival resides in the anatomical location of those cells, i.e. the normal cells are located near the periphery of the tumour and are closely related to the blood vessels, thereby minimising the build-up of acidity in the immediate environment thereof. Hyperthermia has been found to interact synergistically with ionising radiation treatment and chemotherapy, a factor which augments its clinical utility as an anti-cancer treatment modality.

The major obstacle impeding the widespread clinical utilisation of hyperthermia in treating carcinomata has been the inability to deliver localised hyperthermia. Thus, early experimentation involving exposing an entire body to diathermy at temperatures of about 41°C had evidenced a temporary regression of tumours, but shortly after the treatment the tumours began to grow rapidly again.

Attempts have been made to localise heating in the tumour-containing area with a minimum deleterious effect upon adjacent normal tissue by the use of means, such as electromagnetic fields, e.g. lasers and microwaves, and radio frequency (RF)-induced magnetic fields. The latter has been the subject of several publications, for example: "Selective Inductive Heating of Lymph Nodes", Gilchrist *et al., Annals of Surgery, 146,* No. 4, pages 596—606, September, 1957; "Controlled Radio-Frequency Generator for Production of Localised Heat in Intact Animal", Medal *et al., American Medical Association Archives of Surgery, 79* pages 83—87, September, 1959; and "Selective Heating and Cooling of Tissue in Cancer Chemotherapy", Shingleton *et al., Annals of Surgery, 156,* No. 3, pages 408—416.

Those publications described the implantation of powdered magnetic materials, specifically a magnetic form of iron oxide statedly defined as $Fe_2O_3$, into tissue. These particles became heated as a result of the coupling to the magnetic field by the dielectric and hysteresis loss thereof. Those studies utilised magnetic fields at radio frequencies of from 0.12 to 2 megahertz.

Although initial experiments demonstrated that this method for localising induction heating was operable in destroying metastases, two factors militated against this method being accepted as a useful modality for treating carcinomata. Firstly, the magnetic form of iron oxide is insoluble in body fluids and in substantial concentrations may be toxic to and/or rejected by the body; and, secondly, the normal tissue surrounding the tumour became too hot during the heating operation and was subjected to necrosis. This latter effect was due to dielectric heating, i.e. heating resulting from ionic conductivity of body tissue and fluids.

It has been recognized that more disparate heating between the suscepted region and the surrounding region would occur if the excitation field were of lower frequency. The heating of normal tissue takes place by dielectric heating which is a function of the field of frequency sequared or even higher, depending upon the loss tangent, while magnetic hysteresis heating varies linearly with field frequency. Up to the present time, however, no magnetic material has been identified as having the necessary chemical, mechanical and magnetic properties to be useful in contact with animal tissue while permitting the required heating to occur at reduced field frequencies.

The present invention provides a glass, glass-ceramic or sintered ceramic material for use in tumour mass reduction by induction of localised hyperthermia, the material being characterised in that it has a base composition selected from phosphates, silicates and borates and has incorporated therein magnetic iron-containing crystals or magnetite or a ferrite selected from lithium, cobalt, nickel, manganese and barium ferrite, the crystals being of such size, composition, concentration and magnetic properties that, upon exposure to a radio frequency magnetic field not exceeding 10 kilohertz, a coercive force of at least 200 oersteds is imparted to the material and localised heating is induced by essentially only magnetic hysteresis heating.

These materials exhibit high magnetisation, high coercive force and high loop squareness.

Such materials provide means for locally heating tumours in animal tissue to temperatures within the range of from 41 to 50°C, depending upon the time of exposure, such that the malignant cells are preferentially destroyed with essentially no damage or toxic effect upon adjacent normal tissue.

The materials according to the present invention preferably comprises by weight on the oxide bases, from 10 to 70% $Fe_2O_3$, from 10 to 60% $P_2O_5$, between 50 and 90% $Fe_2O_3+P_2O_5$, from 0 to 25% $Li_2O$, from 0 to 25% $SiO_2$, from 0 to 20% $Al_2O_3$, from 0 to 60% $B_2O_3$ and from 0 to 25% $MgO$.

Up to 25% $CoO$, up to 25% $NiO$ and up to 25% $MnO$ may also be present. They may be produced by a process which comprises compounding and heating appropriate components.

The iron-containing crystals incorporated in the present materials preferably have diameters in excess of $5\times10^{-6}$ cms.

The present materials may be derivatised with tumour-specific ions or with antibodies and/or other similarly bioactive molecules directed against a tumour. Also, they may be pre-sensitised to have an affinity for a tumour species.

The present invention also provides a composition which comprises a material in accordance with the present invention and a sterile, non-toxic carrier.

The above-mentioned local heating of tumours may be achieved by localised magnetically-coupled, RF-included hyperthermia medicated by a material which is non-toxic to and, preferably, inert to or compatible with animal tissue and which has incorporated therewithin magnetic field suscepting crystals of certain size, composition, concentration and magnetic properties. A magnetic field is utilised having a sufficiently low frequency that dielectric heating effects are reduced to a negligible level. The magnetic properties are such as to maximise the hysteresis loss, i.e. the material exhibits high magnetisation, high coercive force and high loop squareness, each of those characteristics contributing to hysteresis heating. Those magnetic properties are also constituent with the practical available field induced within simple induction coil configurations. The frequency of the magnetic field is maintained at a sufficiently low level that essentially only magnetic hysteresis heating may occur. Hence, the frequency of the magnetic field is 10 kilohertz or less. The dielectric loss of animal tissue and fluids is small in this low frequency range thereby minimising heating in the absence of a magnetic susceptor. The present materials will promote effectiveness magnetic hysteresis heating with no noticeable body rejection. This enhanced heating effect is due to the use of high concentrations of matrix material, this matrix material being essentially free from any toxic or inflammatory effect upon the animal body. Hence, the magnetic component is encapsulated in a matrix which is inert to or biocompatible with the animal body.

Iron-containing crystals have been determined to be the most desirable magnetic field susceptor material. Certain organic plastics, e.g. Teflon (Registered Trade Mark FTP), have the necessary inertness and no-toxicity for useful matrix materials.

However, compositions selected from glasses, glass-ceramics and sintered ceramics which are non-toxic to and, preferably, compatible with animal tissue, and including in the structure thereof magnetic crystals of iron-containing compounds, have been determined to constitute the most desirable target materials. It will be recognised that the effectiveness and efficiency of the materials are dependent upon the ability thereof to translate magnetic energy into thermal energy, this ability being related to various factors, such as crystal type and concentration and the presence of pre-crystalline or semi-amorphous regions. Consequently, it will be apparent that bodies containing substantial quantities of ferrimagnetic crystals will be preferred. In general, the magnetic iron-containing crystals will consist of magnetite ($Fe_2O_3$) or a solid solution ferrite. Moreover, in as much as the coercive force exhibited by the crystal phase varies with the size of the crystals, laboratory experience has indicated that the crystals should have a diameter in excess of $5\times10^{-6}$ cms (500 Å), (to exceed superparamagnetic size) and, preferably, at least $1\times10^{-4}$ cms (10,000 Å) 1 micron) to produce domain wall motion.

Customarily, the animal tissue would be heated to temperatures within the range of from 41 to 44°C in as much as those temperatures would cause necrosis of tumour tissue. In contrast, normal animal tissue is not destroyed until temperatures of about 48°C are reached. It has been found, however, that brief exposures to temperatures up to 50°C may be tolerated with very little destruction of normal tissue. Such higher temperatures quickly destroy tumour cells so that the duration of the treatment may be significantly reduced. Hence, a series or pulses of RF magnetic energy may be utilised; the time of each pulse effective to include necrosis of tumour tissue with virtually no effect upon normal tissue may be determined empiracally. The concentration of material necessary to induce the desired heating effect may also be determined empiracally. Thus, the upper temperature of heating may be controllably limited by suitably selecting the material and regulating the quantity thereof administered.

Glasses and glass-ceramics of diverse base constituents, e.g. silicates, aluminosilicates, boro-silicates, borates and phosphates, and containing iron oxide in significant amounts are known. When batch materials for such glasses are melted under oxidising or neutral conditions, the resulting glasses may exhibit magnetic behaviour, the magnitude of the behaviour being a function of, for example, glass composition, annealing schedule and presence of a minor amount of magnetic crystallisation. When the glass melts are appropriately quenched or glass bodies subsequently exposed to the proper heat treatment, minute crystals structurally similar to magnetite may be developed and/or caused to grow in size within the glassy matrix and the ferromagnetic behaviour then evidenced by the bodies is substantially enhanced. Glass-ceramic bodies are generally highly crystalline, i.e. greater than 50%, by volume, crystalline.

United States Patent No. 3,193,503 discloses the production of glass-ceramic articles consisting essentially, expressed in weight percent on the oxide basis, of from 16 to 50% MgO, from 37 to 60% $Fe_2O_3$, from 20 to 45% $SiO_2$ and from 0 to 15% of mineralisers or nucleants, such as $CaF_2$, CoO, NiO, $V_2O_5$, $MoO_3$ and $ThO_2$. The resultant articles were termed "magnetic ceramic ferrites", but no crystallisation identification data were supplied.

United States Patent No. 3,694,360 describes the production of glass-ceramics demonstrating ferrimagnetic properties. The compositions operable therefor consist essentially, expressed in parts, by weight, on the oxide basis, of from 35 to 55 $Fe_2O_3$, from 5 to 15 $Li_2O$, from 10 to 50 $SiO_2$ and from 1 to 15 ZnO. The predominant crystal phase is stated to comprise a lithium ferrite.

United States Patent No. 3,492,237 discusses glass-ceramic bodies having compositions within the $Li_2O-Na_2O-Al_2O_3-Fe_2O_3-SiO_2$ system wherein lithium ferrite is a primary crystal phase. The operable formulations have a mole ratio of $SiO_2:Na_2O:Al_2O_3$ of from 11 to 13: from 3 to 4: from 4 to 1 with from 1 to 10 moles each of $Fe_2O_3$ and $Li_2O$ per mole of $Al_2O_3$.

United States Patent No. 4,140,645 reports glasses and glass-ceramics containing crystals of $Fe_3O_4$ with, optionally, a transition metal ferrite, for example, cobalt ferrite and nickel ferrite. The operable glass compositions are categorised into two groups, expressed in terms of weight percent on the oxide basis:

(a) from 2 to 10% $Na_2O$ and/or $K_2O$, from 5 to 20% $B_2O_3$, from 15 to 40% FeO, from 0 to 32% $Al_2O_3$ and from 35 to 65% $SiO_2$; and

(b) from 1.5 to 6% $Li_2O$, from 10 to 40% FeO, from 10 to 20% $Al_2O_3$, from 45 to 66% $SiO_2$, from 0 to 5% $TiO_2$ and/or $ZrO_2$ and from 0 to 5% $B_2O_3$, at least 1% $B_2O_3$ being required when the proportion of FeO is less than 15%. Likewise, the operable glass-ceramic compositions are formulated from two groups, expressed in terms of weight percent on the oxide basis:

(a) from 2 to 10% $Na_2O$ and/or $K_2O$, from 5 to 20% $B_2O_3$, from 15 to 40% FeO, from 15 to 32% $Al_2O_3$ and from 35 to 50% $SiO_2$; and

(b) from 1.5 to 6% $Li_2O$, from 10 to 40% FeO, from 10 to 20% $Al_2O_3$, from 45 to 66% $SiO_2$, from 0 to 5% $TiO_2$ and/or $ZrO_2$ and from 0 to 5% $B_2O_3$, at least 1% $B_2O_3$ being required when the proportion of FeO is less than 15%.

Both the base glass and glass-ceramic compositions spontaneously precipitate $Fe_3O_4$ when the molten batches are cooled to a glass body. Subsequent heat treatment of the glass bodies gives rise to the *in situ* growth of silicate crystals, e.g. mullite, beta-quartz solid solution and beta-spodumene solid solution, yielding a highly-crystalline glass-ceramic body. The $Fe_3O_4$ crystals may experience some grain growth during that heat treatment.

It will be appreciated that, as a matter of convenience, the above references report the total iron oxide content of the cited materials, customarily present as a combination of FeO and $Fe_2O_3$, as either "FeO" or "$Fe_2O_3$". Hence, in the interest of simplicity and because analysis of the individual proportions of FeO and $Fe_2O_3$ is tedious and knowledge of the precise content of each is unnecessary, the full amount of the iron oxide present was expressed as either "FeO" or "$Fe_2O_3$".

Each of the above references was directed to base glasses in the silicate system. Such compositions are operable in accordance with the present invention. However, while there have been publications of silicate-based glasses suitable for bonding to bone or other living tissue e.g. United States Patent No. 4,159,358 describing glasses consisting essentially expressed in terms of weight percent on the oxide basis, of from 40 to 60% $SiO_2$, from 10 to 32% $Na_2O$, from 10 to 32% CaO, from 0 to 18% $CaF_2$, from 0 to 20% $B_2O_3$ and from 0 to 12% $P_2O_5$, it has long been recognised that, in general, phosphate-based glasses are more compatible with living tissue than are silicate-based compositions. Consequently, the preferred base glasses, glass-ceramics and/or sintered ceramics according to the present invention have compositions founded in the phosphate composition system.

In the application of the present materials to tumour mass reduction, the desired localised hyperthermia may be achieved in numerous ways. For example, an aqueous dispersion of the target material in very finely-divided form may be injected directly into a tumour and/or into normal tissue immediately adjacent to the tumour. Subsequent exposure by the defined RF induced magnetic field causes the target material to be heated.

Alternatively an aqueous dispersion of the powdered material may be injected via intravenous or arterial routes at a site near or distal to the tumour. Blood flow acts to transport the material to the site of the tumour. Assistance in localising the injected magnetic material utilising this method may be found by guiding the passage with a magnet.

In the case of surgical exposure of a tumour, the target material may be injected into or applied to the outside of the tumour. Hyperthermia may be induced by magnetic field induction heating before and/or after the incision has been closed. Thus, the treatment may consist of a series of exposures. The material will desirably be inert or else harmlessly degraded by body fluids, the degradation occurring slowly enough to permit the material to be at the tumour site for a substantial period. Hence, a succession of individual treatments with RF fields to secure localised heating may be conducted with only one implacement of mediating material.

It is possible to derivatise the target particles with tumour specific ions or with antibodies and/or other similarly bioactive molecules directed against the tumour, thereby causing specific localisation of the material in and/or around the tumour. Thus, agents specific to a particular tumour may be attached directly to the material or by the use of chelating or other coupling agents.

The physical properties of tumours may also be utilised in localising the target material in the areas thereof. For example, tumours generally exhibit pH values either in the range of from 3 to 4 or in excess of 8.5. The pH of normal body fluids is about 7.4.

Yet another method contemplates pre-sensitising the material to have affinity for a tumour species and thereafter delivering the material to the tumour site by, for example, injection, cannulation or magnetic guidance. Such pre-sensitisation may involve the surface of the material or the bulk thereof. For example, a ceramic may be etched and the pores filled with the sensitising agent. Illustrations of such sensitising agents include gallium for lung carcinomata and K/Mg for low pH tumours.

Table 1 below records several glass compositions, analysed in terms of weight percent on the oxide basis, operable in accordance with the present invention. The actual batch ingredients therefor may comprise various materials, either oxides or other compounds, which, when melted together with the other components, will be converted into the desired oxide in the proper proportions.

The batch ingredients were compounded, ball-milled together to assist in achieving a homogeneous melt and the mixture charged into silica, porcelain, MgO or platinum crucibles. The crucibles were introduced into a furnace operating at from 1300 to 1550°C and the batch ingredients melted together for about one hour. Thereafter, the melts were poured onto a water cooled steel mould and the melt quenched by a steel platen being immediately placed into contact with the top surface thereof. (In Samples 5 to 11 all the iron is reported in terms of $Fe_2O_3$.)

## TABLE 1

|  | 1 | 2 | 3 | 4 | 5 | 6 |
|---|---|---|---|---|---|---|
| $Fe_2O_3$ | 55.0 | 31.4 | 36.1 | 36.2 | 34.4 | 19.3 |
| FeO | 4.4 | 11.5 | 18.3 | 12.8 | — | — |
| $P_2O_5$ | 23.7 | 21.9 | 22.9 | 18.8 | 37.0 | 47.4 |
| $Li_2O$ | 11.6 | 10.2 | — | 7.8 | 15.3 | 19.2 |
| $SiO_2$ | 3.4 | 13.3 | 6.2 | 11.9 | 10.1 | — |
| $Al_2O_3$ | 0.4 | 11.5 | 5.5 | 11.0 | 0.26 | 12.9 |
| MnO | — | — | 10.4 | — | — | — |
| $B_2O_3$ | — | — | — | 2.0 | — | — |
| MgO | — | — | — | — | 2.38 | 0.25 |

## TABLE 1 (Cont'd)

|  | 7 | 8 | 9 | 10 | 11 |
|---|---|---|---|---|---|
| $Fe_2O_3$ | 31.0 | 35.0 | 40.5 | 35.4 | 30.0 |
| $P_2O_5$ | 30.5 | 26.6 | 18.4 | 26.3 | 26.6 |
| $Li_2O$ | 14.4 | 14.4 | 0.08 | 14.4 | 12.0 |
| $SiO_2$ | 18.8 | 20.0 | 10.8 | 14.2 | 16.6 |
| $Al_2O_3$ | 0.66 | 1.23 | 0.15 | 0.14 | 13.7 |
| MnO | — | — | 18.1 | — | — |
| $B_2O_3$ | — | — | — | 4.84 | — |
| MgO | 4.68 | 4.46 | 10.3 | 5.46 | 2.81 |

No $Al_2O_3$ was present in the batch materials for Samples 1, 5 and 7 to 10, no $Li_2O$ was batched in Sample 9 and no MgO was included in the batch of Sample 6. The analysed values recorded of those components constitute impurities which were most likely picked up from the crucibles during melting.

The melts crystallised upon cooling and X-ray diffraction analyses were conducted upon the resultant products of Samples 1 to 4. Sample 1 appeared to be almost completely crystallised, the major phase being hematite ($Fe_2O_3$) with a minor amount of what is believed to be a lithium iron phosphate. The pattern of the latter phase did not identically match any published standard. Sample 2 exhibited substantially more vitreous phase and the crystals appeared to consist of about 60% lithium ferrite (believed to have the stoichiometry of $LiFe_3O_8$) and 40% of what has been conjectured to be a lithium-doped magnetite. Thus, a slight shift in the $Fe_3O_4$ peak was observed. Sample 3 also contained a significant amount of glassy phase with the bulk of the crystals demonstrating a diffraction pattern very close to that of magnetite. The presence of manganese in the starting materials suggests the possibility of manganese-doped magnetite or a small amount of manganese ferrite. Sample 4 appeared to have a microstructure similar to that of Sample 2, the crystal phase consisting primarily of lithium ferrite and the postulated lithium-doped magnetite. It has been hypothesised that $B_2O_3$ may be substituted in part for $Li_2O$ in the crystal phase. When placed in a magnetic field, Sample 2 demonstrated the greatest activity followed by Sample 4, Sample 3 and Sample 1, in that order.

Table 2 below reports qualitative measurements of magnetism demonstrated by the exemplary compositions of Table 1 along with a determination of the magnetisation exhibited by those compositions in a field of 700 oersteds ($M_{700}$). A measurement of the heating value (calories/loop/gram), as determined from the area under the hysteresis loop, is tabulated for Samples 5 to 11. Furthermore, a qualitative judgement of the squareness of the hysteresis loop is provided for Samples 5 to 11.

TABLE 2

| Sample | Magnetism | $M_{700}$ | Cal./Loop/Gram | Squareness |
|---|---|---|---|---|
| 1 | | 2.9 | | |
| 2 | | 20 | | |
| 3 | | 18 | | |
| 4 | | 21 | | |
| 5 | Very slight | 0.42 | $1.76 \times 10^{-6}$ | Poor |
| 6 | Slight | 0.67 | $1.48 \times 10^{-6}$ | Good |
| 7 | Slight | 2.2 | $3.2 \times 10^{-6}$ | Very good |
| 8 | Strong | 6.2 | $2.48 \times 10^{-5}$ | Fair |
| 9 | Very strong | 8.7 | $4.36 \times 10^{-5}$ | Fair |
| 10 | Strong | 7.3 | $1.03 \times 10^{-5}$ | Excellent |
| 11 | Very strong | 13.26 | $2.0 \times 10^{-4}$ | Fair-good |

In general, the presently preferred compositions will consist essentially, by weight as analysed on the oxide basis, of from 10 to 70% $Fe_2O_3$, from 10 to 60% $P_2O_5$, between 50 and 90% $Fe_2O_3+P_2O_5$, from 0 to 25% $Li_2O$, from 0 to 25% $SiO_2$, from 0 to 20% $Al_2O_3$, from 0 to 60% $B_2O_3$ and from 0 to 25% MgO. Where a ferrite crystal phase is desired, up to 25% of metal oxides, such as CoO, NiO and MnO, may be included.

In the following studies illustrating the present invention, particles of Sample 1 comprised the magnetic ceramic material and murine adenocarcinoma of the breast BW10232 was utilised as the tumour. Sample 1 was milled to particles having an average size of $2 \times 10^{-4}$ cms (2 microns) or less and exhaustively washed in isotonic Dulbeccos phosphate buffered saline (DPBS), pH 7.2, to remove possible toxic by-products of the production and milling processes. Murine adenocarcinoma arose spontaneously in the mammary gland of a C57BL/6J (B6) inbred mouse at the Jackson Laboratories, Bar Harbour, Maine, U.S.A. in 1958. Gross examination of the tumour reveals a soft, white, encapsulated mass with frequent hemorrhagic zones. The tumour is palpable from 7 to 10 days post inoculation of tumour brei (minced, finely-divided tissue with variable proportions of single cell and clumped cellular masses).

Cryo-preserved tumour having a volume of about 1 $cm^3$ was obtained from the National Cancer Institute Division of the Cancer Treatment Contract Production Facility, Mason Research Institute, Worcester, Massachusetts, USA. The tumour was stored at $-209°C$ in a cryo-preservative medium and shipped in dry ice ($-78°C$). The tumour was briefly held at $-89°C$ in a mechanical freezer until passed.

6

On the day of passage, the cryo-preserved tumour was quick-thawed by immersing the frozen ampoule into a beaker of distilled water at 37°C. The tumour brei was asceptically transferred to sterile tissue culture Petri dishes and minced with sterile surgical scalpel blades. All manipulations were performed within a laminar flow containment hood recommended by the National Cancer Institute for oncogenic agents of undefined hazard. The finely-minced tumour was brought to a final volume of 1.5 ml with medium 199 (Grand Island Biological Company, Grand Island, New York U.S.A.), 15% C57BL/J6 normal serum and brought through a series of decreasing diameter syringe needles ($18° \rightarrow 20° \rightarrow 23° \rightarrow 27°$) until the tumour could be injected through a 27° tuberculin syringe. The initial subcutaneous inoculum (from 5 to 10% suspension) was divided equally between six C57BL/6J age-matched male mice (Jackson Laboratory, Bar Harbour, Maine, U.S.A.). In approximately 10 days, a 1 cm³ mass was crossly apparent in two of the six recipients.

Variable-sized tumours were asceptically dissected free from normal fascia, debrided of necrotic tissue and washed in DPBS. The tumour was thereafter minced with sterile surgical scalpel blades, the brei diluted to an approximate 10% suspension with DPBS and passaged through decreasing diameter syringes as described above. Analyses of the single cell population employing a $1.4 \times 10^{-2}$ cms (140 micron) orifice and a Coulter ZBI-H-4 channelyser (Coulter Electronics, Hialeah, Florida, U.S.A.) calibrated with $1 \times 10^{-3}$ cms (10 micron) spheres indicated a skewed population range from 20 to 380 µm³ with the majority of the cells (~55%) being greater than 80 µm³. 0.2 ml of the 10% tumour suspensions ($1 \times 10^7$ of 80 µm³ or larger cells) were inoculated subcutaneously in the inguinal region. The animals were closely monitored for appearance of tumour foci by palpation. Tumour cells not used for passage were resuspended in cryo-preservative medium (medium 199, 15% C57BL/6J normal serum, 10% DMSO) and refrozen to −80°C. Tumours so frozen were fully capable of *in situ* growth when passaged after thawing.

The following is a general discussion of the experimental protocol performed in these studies. C57BL/6J male age-matched mice were inoculated subcutaneously with 0.2 cc of a 10% tumour preparation in the left and right inguinal region. In most instances, a single left and right tumour focus developed and the present treatment was initiated when the tumours approached 4 or 5 mm in diameter (54 to 65 mm³ volume). The left inguinal region immediately adjacent to the base of the tumour was subcutaneously injected with the ceramic suspension. Injection of the ceramic was conducted very slowly to avoid excessive pressure build-up within the tissue. One injection site only was utilised in most cases because multiple injections tended to displace previously-injected ceramic out through the initial injection site. Multiple injections may be used if initial injection volumes are small. The right tumour received no ceramic, but was exposed to a RF magnetic field. This action permitted the tumour-bearing animal to serve as both experimental and control.

Prior to exposure to a RF magnetic field, surface temperatures of the inguinal tumour receiving ceramic and the axillary region were measured utilising a calibrated microthermistor. The mice were then placed inside a "Plexiglass"® restrainer and positioned within a ten-turn, water-cooled solenoidal induction coil having a diameter of 9 cms (3.5″) and a height of 20 cms (8″). A 30 KW GCCO motor generator was utilised to drive the coil at 10 kilohertz. The unit was capable of supplying up to 1400 oersteds within the coil. The mice were treated for five minutes at 10 kilohertz and 700 oersteds and thereafter removed from the restrainer. The surface temperatures of the treated tumour and the axilla or untreated (right) tumour were measured. In most instances, the mice received only one ceramic injection and one exposure to the magnetic field. The animals were monitored daily with tumour diameters being ascertained by means of a vernier caliper. In a few experiments, only one inguinal tumour was carried and the mice were randomly divided into ceramic treatment alone, ceramic plus RF magnetic exposure, or no treatment.

Table 3 below illustrates the localised heating effect caused by the RF magnetically coupled induction of the subcutaneously implanted particles of Sample 1. Hence, the average tumour surface temperature before treatment was 36.3°C, while immediately after treatment the average temperature was measured as 40.3°C. In contrast, when the axillary temperatures before and after treatment were measured, no significant increase in temperature was observed. Accordingly, these data clearly attest to the capability of magnetic ceramic materials to induce localised hyperthermia. Although the recorded surface temperature measured did not attain the preferred clinical hyperthermia range of from 42.5 to 43°C, the temperature of tissue closer to the ceramic particles was unquestionably higher than 40.5°C because of the tumour regression observed and described below.

Five C57BL/6J mice evidencing a single left inguinal tumour averaging about 8.2 mm in diameter were injected with 0.28 grams of Sample 1 particles at the base of the tumour following the above protocol. Four animals, *viz.* a, b, c and d, received a total body exposure to a RF magnetic field of 700 oersteds at a frequency of 10 kilohertz for five minutes while the fifth mouse, e, served as a ceramic-only control. Animals a and b (containing particles of Sample 1 and being exposed to a RF magnetic field) exhibited growth after 13 days in excess of that demonstrated by e, the control sample not treated. However, animals c and d (containing particles of Sample 1 and being subjected to a RF magnetic field) demonstrated about a two-fold reduction in tumour volume on days 7 and 8 relative to animal e. To ascertain whether the observed diminution of growth rate was a reproducible result of the ceramic+RF magnetic field therapy, the double tumour bearing experimental protocol described above was performed.

Five C57BL/6J mice, *viz,* f, g, h, i and j, bearing single left and right inguinal tumours having an average diameter of about 5 mm were subcutaneously injected with 0.3 grams of Sample 1 particles at the base of

7

the left tumour only. After this injection the entire bodies of f, g, h and i were exposed for five minutes to a 700 oersted RF magnetic field at a frequency of 10 kilohertz. Thus, animal j, containing ceramic particles, but not treated, served as the control. In animals f, g and h, the left tumour mass grew at a diminished rate relative to the non-treated right tumour. Animal h represented particularly interesting data since no detectable tumour was present on the right side at the time of RF exposure, but after several days a focus appeared which rapidly outgrew the treated left side. Animal i responded poorly to treatment although gross examination after seven days revealed a reduced left (treated) mass when compared to the right (untreated) mass. Examinations of the tumours in control animal j manifested essentially equivalent growth on both the left and right sides.

Eight days after treatment the animals were sacrificed and careful gross dissection of the mice was undertaken. An appreciable accumulation of ceramic was noted in the treated tumour area with little or no observable spreading. This latter finding was of especial importance since it indicated that the RF exposure may be effectively reapplied on subsequent occasions after the initial injection of ceramic particles.

The final weights of the tumours dissected clear of normal tissue and ceramic were measured. The data reported in Table 4 below clearly illustrate a tumour mass reduction factor (left side v. right side) ranging from 1.81 to 4.81. Control animal j demonstrated similar left and right tumour masses with a ratio of 0.91.

Examination of the normal tissue immediately adjacent to the ceramic particles manifested essentially no evidence of necrosis resulting from the ceramic-mediated, magnetic field treatment.

TABLE 3

| Animal | Ceramic dose | Surface temperature · before treatment (°C) | | Surface treatment after treatment (°C) | |
|---|---|---|---|---|---|
| | | tumour | axilla | tumour | axilla |
| a | 0.28 g | 36 | 37 | 42.5 | 37 |
| b | 0.28 g | — | — | 40.5 | — |
| f | 0.30 g | 36 | 36.5 | 40 | 37 |
| g | 0.30 g | 37 | 37 | 38.5 | 38.5 |
| h | 0.30 g | 37 | 37 | 43 | 38 |
| i | 0.30 g | 35.5 | 36.5 | 37.5 | 36.5 |

TABLE 4

| Animal | Left tumour mass (g) | Right tumour mass (g) | Ratio right tumour mass:left tumour mass |
|---|---|---|---|
| f | 1.65 | 3.35 | 2.03 |
| g | 2.20 | 4.0 | 1.82 |
| h | 0.80 | 3.85 | 4.81 |
| i | lost* | lost* | — |
| j | 3.5 | 3.2 | 0.91 |

* Animal dies on seventh day. Gross observation on seventh days indicated an approximate two-fold difference between left and right tumour masses.

The above studies illustrating the absence of migration of the ceramic particles from the site of the subcutaneous injection, coupled with the excellent tumour mass reductions observed after a single five-minute RF exposure, clearly suggested that total tumour eradication could be possible where exposure times are increased and/or multiple treatments are applied. Moreover, a more highly magnetic ceramic would require less time to heat the tumour tissue to the hyperthermia range. The effectiveness of the ceramic may also be improved by increasing the coercive force thereof by the addition of doping ions, such as cobalt, into the ferritic structure.

In a single experiment with a C57BL/6J mouse utilising the above protocol, 0.3 grams of Sample 1 were injected intra-tumour and subcutaneously at the margins of an inguinal tumour. The body of the animal was then subjected for one hour to a 700 oersted field having a frequency of 10 kilohertz. Subsequent examination indicated essentially total eradication of the tumour mass, thereby substantiating the above hypothesis that total tumour extirpation is feasible in accordance with the present invention. Further experimental work has demonstrated that administering pure uncapsulated magnetic susceptor materials, e.g. magnetite, at one-half the dosage level utilised with the present materials produced gross inflammation and obvious rejection.

**Claims**

1. A glass, glass-ceramic or sintered ceramic material for use in tumour mass reduction by induction of localised hyperthermia, the material being characterised in that it has a base composition selected from phosphates, silicates and borates and has incorporated therein magnetic iron-containing crystals of magnetite or a ferrite selected from lithium, cobalt, nickel, manganese and barium ferrite, the crystals being of such size, compositions, concentration and magnetic properties that, upon exposure to a radio frequency magnetic field not exceeding 10 kilohertz, a coercive force of at least 200 oersteds is imparted to the material and localised heating is induced by essentially only magnetic hysteresis heating.

2. A material as claimed in claim 1 characterised in that it comprises, by weight on the oxide bases, from 10 to 70% $Fe_2O_3$, from 10 to 60% $P_2O_5$, between 50 and 90% $Fe_2O_3+P_2O_5$, from 0 to 25% $Li_2O$, from 0 to 25% $SiO_2$, from 0 to 20% $Al_2O_3$, from 0 to 60% $B_2O_3$ and from 0 to 25% MgO.

3. A material as claimed in claim 1 or claim 2 characterised in that it comprises, by weight on the oxide bases, from 0 to 25% CoO, from 0 to 25% NiO and from 0 to 25% MnO.

4. A material as claimed in any of claims 1 to 3 characterised in that the iron-containing crystals have diameters in excess of $5\times10^{-6}$ cms.

5. A material as claimed in any of claims 1 to 4 characterised in that it is derivatised with tumour-specific ions or with antibodies and/or other similarly bioactive molecules directed against a tumour.

6. A material as claimed in any of claims 1 to 5 characterised in that it is pre-sensitised to have an affinity for a tumour species.

7. A composition characterised in that it comprises a material as claimed in any of claims 1 to 6 and a sterile, non-toxic carrier.

**Patentansprüche**

1. Glas-, glas-keramisches- oder gesintertes keramisches Material zur Verwendung bei der Tumormassenreduktion durch Induktion lokalisierter Hyperthermie, dadurch gekennzeichnet, daß das Material eine Grundzusammensetzung aufweist, ausgewählt aus Phosphaten, Silikaten und Boraten und daß hierin magnetische eisenhaltige Kristalle aus Magnetit oder einem Ferrit, ausgewählt aus Lithium-, Kobalt-, Nickel-, Mangan- und Bariumferrit, inkorporiert sind, wobei die Kristalle eine solche Gestalt, Zusammensetzung, Konzentration und magnetische Eigenschaften aufweisen, daß wenn sie einem Hochfrequenz-magnetischen Feld von nicht über 10 Kilohertz ausgesetzt werden, dem Material eine Koerzitivkraft von wenigstens 200 Oersted verliehen wird, und eine lokalisierte Erhitzung im wesentlichen nur durch magnetische Hysterese-Erhitzung induziert wird.

2. Material nach Anspruch 1, dadurch gekennzeichnet, daß es—in Gewichtsprozent auf Oxidbasis—10 bis 70% $Fe_2O_3$, 10 bis 60% $P_2O_5$, zwischen 50 und 9% $Fe_2O_3+P_2O_5$, 0 bis 25% $Li_2O$, 0 bis 25% $SiO_2$, 0 bis 20% $Al_2O_3$, 0 bis 60% $B_2O_3$ und 0 bis 25% MgO umfaßt.

3. Material nach Anspruch 1 oder 2, dadurch gekennzeichnet, daß es—in Gewichtsprozent auf Oxidbasis—0 bis 25% CoO, 0 bis 25 NiO und 0 bis 25% MnO umfaßt.

4. Material nach einem der Ansprüche 1 bis 3, dadurch gekennzeichnet, daß die eisenhaltigen Kristalle Durchmesser von über $5\times10^{-6}$ cm aufweisen.

5. Material nach einem der Ansprüche 1 bis 4, dadurch gekennzeichnet, daß es mit tumorspezifischen Ionen oder mit Antikörpern und/oder anderen ähnlichen bioaktiven Molekülen, die gegen einen Tumor wirken, derivatisiert ist.

6. Material nach einem der Ansprüche 1 bis 5, dadurch gekennzeichnet, daß es derart präsensibilisiert ist, daß es eine Affinität für Tumorspezies aufweist.

7. Zusammensetzung, dadurch gekennzeichnet, daß sie ein Material, wie es in einem der Ansprüche 1 bis 6 charakterisiert ist und einen sterilen, nicht-toxischen Träger umfaßt.

**Revendications**

1. Une matière en verre, en virocéramique ou en céramique frittée pour utilisation dans la réduction de la masse d'une tumeur par induction d'hyperthermie localisée, cette matière étant caractérisée en ce qu'elle a une composition de base choisie parmi les phosphates, les silicates et les borates et a, incorporés en son sein, des cristaux magnétiques, contenant du fer, de magnétite ou d'une ferrite choisie parmi les ferrites de lithium, de cobalt, de nickel, de manganèse et de baryum, ces cristaux étant d'une grosseur, d'une composition, d'une concentration et ayant des propriétés magnétiques telles que, lors d'une exposition à un champ magnétique à haute fréquence n'excédant pas 10 kilohertzs, une force coercitive d'au moins 200 oersteds est impartie à la matière et qu'un échauffement localisé est induit essentiellement seulement par chauffage par hystérésis magnétique.

2. Une matière selon la revendication 1, caractérisée en ce qu'elle comprend, en poids sur la base des oxydes, de 10 à 70% de $Fe_2O_3$, de 10 à 60% de $P_2O_5$, entre 50 et 90% de $Fe_2O_3+P_2O_5$, de 0 à 25% de $Li_2O$, de 0 à 25% de $SiO_2$, de 0 à 20% d'$Al_2O_3$, de 0 à 60% de $B_2O_3$ et de 0 à 25% de MgO.

3. Une matière selon la revendication 1 ou 2, caractérisée en ce qu'elle contient, en poids sur la base des oxydes, de 0 à 25% de CoO, de 0 à 25% de NiO et de 0 à 25% de MnO.

4. Une matière selon l'une quelconque des revendications 1 à 3, caractérisée en ce que les cristaux contenant du fer ont des diamètres supérieurs à $5\times10^{-6}$ cm.

5. Une matière selon l'une quelconque des revendications 1 à 4, caractérisée en ce qu'elle est sous la forme de dérivés avec des ions spécifiques à la tumeur ou avec des anticorps et/ou d'autres molécules similairement bioactives dirigées contre une tumeur.

6. Une matière selon l'une quelconque des revendications 1 à 5, caractérisée en ce qu'elle est pré-sensibilisée pour avoir une affinité pour une espèce de tumeur.

7. Une composition caractérisée en ce qu'elle comprend une matière selon l'une quelconque des revendications 1 à 6 et un véhicule non-toxique stérile.